# EUROPEAN PATENT APPLICATION

(11) **EP 2 816 100 A1**
(43) Date of publication of application: **24.12.2014**
(21) Application number: 13749910.9
(22) Date of filing: 15.02.2013
(51) Int. Cl.: C11B 9/00, A23L 1/221, A23L 1/30, A23L 2/00, A61K 8/44, A61K 8/49, A61Q 11/00, A61Q 13/00

(54) **AGENT FOR IMPROVING AROMA INTENSITY AND/OR AROMA QUALITY**

(30) Priority: 15.02.2012 JP 2012030216
(71) Applicant: Takasago International Corporation, Tokyo 144-8721 (JP)
(72) Inventor: TOYOHARA Yoshikazu, Hiratsuka-shi Kanagawa 254-0073 (JP); HIRAMOTO Tadahiro, Hiratsuka-shi Kanagawa 254-0073 (JP); OMOTANI Naoto, Hiratsuka-shi Kanagawa 254-0073 (JP)
(74) Representative: Zimmermann & Partner
(86) International application number: PCT/JP2013/053746
(87) International publication number: WO 2013/122226

(57) **Abstract**

The present invention provides an agent for enhancing aroma intensity and/or aroma quality of a flavor composition to be added to a food, drink, pharmaceutical product, oral care product, or the like, the agent comprising γ-aminobutyric acid and naringenin, as well as a method for enhancing aroma intensity and/or aroma quality of the flavor composition.

## Description

### Technical Field

The present invention relates to an agent for enhancing aroma intensity and/or aroma quality of a flavor composition to be added to a food, drink, pharmaceutical product, oral care product, or the like, and a method for enhancing aroma intensity and/or aroma quality of the flavor composition.

### Background Art

Various flavor compositions have been used for enhancing and improving the flavor and aroma of foods, drinks, pharmaceutical products, oral care products, and the like. In particular, to differentiate a product from others, the product is desirably excellent in the intensity of a perceived aroma as well as the growth and persistence of the aroma, and there is a demand for a novel method which enables enhancement of aroma intensity and aroma quality of a flavor composition.

Japanese Patent Application Publication No. 2010-130978 describes enhancement of the flavor of a fish shavings extract or a fish shavings extract-containing seasoning/condiment by adding β-damascenone, and shows tomato as an example of foods containing β-damascenone. However, the target is limited to fish shavings, and improvement of a flavor by γ-aminobutyric acid and naringenin is not described.

Japanese Patent Application Publication No. 2003-102420 states that an extract of a vegetable selected from aromatic vegetables, tomato, spices, and mushrooms exhibits an excellent effect of reducing the malodor characteristic of starch or fish meat. However, the amount of the extract added is as high as 0.02 to 10 wt% relative to the processed food, and the effect is the reduction of the malodor characteristic of starch or fish meat. Hence, enhancement of an aroma is not described.

Japanese Patent Application Publication No. Hei 09-70277 states that, to reduce the green notes of green bell pepper, carrot or tomato juice is added at 10 to 1000% to green bell pepper juice. However, the amount of the vegetable juice added to the object is extremely large. In addition, since the object is to reduce the green notes of green bell pepper, enhancement of an aroma is not described.

Japanese Patent Application Publication No. Hei 6-339364 states that the unpleasant retort odor can be reduced by adding a flavor oil (spice, vegetable, or seasoning) at 0.1 to 10 wt% to a retort pouch food. However, the amount of the flavor oil added to the food is large. In addition, the object is to remove the retort odor, and enhancement of an aroma is not described. Moreover, neither γ-aminobutyric acid nor naringenin is described.

### Summary of Invention

An object of the present invention is to provide an agent for enhancing aroma intensity and/or aroma quality of a flavor composition to be added to a food, drink, pharmaceutical product, oral care product, or the like, and a method for enhancing aroma intensity and/or aroma quality of the flavor composition.

The inventors of the present invention have found that an excellent effect of enhancing aroma intensity and/or aroma quality of a flavor composition for a food, drink, or the like can be achieved by adding naringenin and γ-aminobutyric acid to the flavor composition under a predetermined condition. This finding has led to the completion of the invention of the present application.

Specifically, the present invention provides an agent for enhancing aroma intensity and/or aroma quality of a flavor composition, the agent comprising γ-aminobutyric acid and naringenin.

The present invention also provides a method for enhancing aroma intensity and/or aroma quality of a flavor composition, the method comprising adding γ-aminobutyric acid and naringenin to the flavor composition.

The present invention also provides a flavor composition which comprises the above-described agent for enhancing aroma intensity and/or aroma quality, or which has aroma intensity and/or aroma quality enhanced by the above-described method.

The present invention also provides a method for enhancing aroma intensity and/or aroma quality of a food, drink, pharmaceutical product, or oral care product, the method comprising the step of adding the above-described flavor composition to the food, drink, pharmaceutical product, or oral care product.

The present invention also provides a food, drink, pharmaceutical product, or oral care product comprising the above-described flavor composition.

The method of the present invention in which γ-aminobutyric acid and naringenin are used in combination under a predetermined condition makes it possible to obtain an improved flavor composition whose aroma intensity and/or aroma quality are enhanced without impairing characteristics of the flavor composition.

### Description of Embodiments

An agent for enhancing aroma intensity and/or aroma quality of the present invention comprises γ-aminobutyric acid and naringenin. Meanwhile, a method for enhancing aroma intensity and/or aroma quality of the present invention comprises adding γ-aminobutyric acid and naringenin to a flavor composition.

In the present invention, "enhancement of aroma intensity" means that the strength of the perceived aroma increases, and "enhancement of aroma quality" means that the growth and/or persistence of the aroma are enhanced.

In the agent for enhancing aroma intensity and/or aroma quality of the present invention, the component ratio of the γ-aminobutyric acid to the naringenin can be determined, as appropriate, by those skilled in the art according to properties of the target flavor composition and the finished product (food, drink, pharmaceutical product, oral care product, or the like) to which the flavor composition is added, as well as the required effect. The component ratio of the γ-aminobutyric acid to the naringenin is preferably in a range from 1:10000 to 100000:1, more preferably in a range from 1:1000 to 10000:1, and further preferably in a range from 1:100 to 1000:1.

In addition, the γ-aminobutyric acid and the naringenin can be added in any forms. For example, each of the compounds itself may be added, or the γ-aminobutyric acid and the naringenin may be added in the state of a natural extract containing both of the components, for example, a processed animal or plant product. When the processed animal or plant product itself has a characteristic aroma, it is preferable to reduce, before use, the aroma of the animal or plant itself by a method such as distillation, adsorbent treatment, or solvent extraction.

In the present invention, the γ-aminobutyric acid and the naringenin are preferably originated from a processed tomato product. A method for obtaining the processed tomato product comprising γ-aminobutyric acid and naringenin can be determined, as appropriate, by those skilled in the art. For example, the method may be direct extraction with a solvent such as methanol, ethanol, acetone, or ethyl acetate from squeezed tomato juice which may be filtered, if necessary, or from commercially available concentrated transparent tomato juice. Here, ethanol is particularly preferable. Specifically, the following method may be employed: 0.3 to 10 times by weight, preferably 0.5 to 2 times by weight of ethanol is mixed with the squeezed juice or concentrated juice of tomato; the mixture is stirred, and allowed to stand; then, the upper one of the two separated layers is recovered. The concentration of the ethanol added depends on the added amount, and, for example, it is preferable to use ethanol with a high concentration of 95% or higher. In addition, the concentration of the squeezed juice or concentrated juice of tomato is preferably high in view of the phase separation. The concentration is, for example, Brix 20 to 80, preferably Brix 30 to 70, and more preferably Brix 50 to 65. In this method, highly water-soluble components can be reduced. Hence, this method is preferable because such a processed tomato product can be added to the flavor composition without causing formation of insoluble matters such as turbidity and deposition. Another employable method is that a solvent such as methanol, ethanol, acetone, or ethyl acetate is added to squeezed juice or concentrated juice of tomato; the mixture is heated to produce a distillate; and then the distillation residue liquid is recovered. Here, ethanol is particularly preferable. Specifically, 0.01 to 10 times by weight, preferably 0.1 to 1 times by weight of ethanol is mixed with the squeezed juice or concentrated juice of tomato, and the mixture can be heated at, for example, 30 to 100°C, and more preferably 50 to 80°C. Since heating at high temperature causes development of unnecessary aromas, coloration, and the like, the heating is preferably conducted at low temperature. In this respect, the heating is preferably conducted under reduced pressure. The squeezed juice or concentrated juice of tomato may be scorched, if the concentration is high. Hence, the concentration may be, for example, Brix 1 to 60, preferably Brix 5 to 50, and more preferably Brix 20 to 40. Since the unnecessary green notes originated from tomato can be reduced by the distillate, this method is preferable for making versatile the processed tomato product to be added to the flavor composition.

The present invention also provides a flavor composition which comprises the above-described agent for enhancing aroma intensity and/or aroma quality, or which has aroma intensity and/or aroma quality enhanced by the above-described method. In the flavor composition of the present invention, the concentration of γ-aminobutyric acid is preferably 0.1 to 10000 ppm, more preferably 0.1 to 1000 ppm, still more preferably 1 to 1000 ppm, and further preferably 10 to 100 ppm, while the concentration of naringenin is preferably 0.01 to 10000 ppm, more preferably 0.01 to 1000 ppm, still more preferably 0.05 to 1000 ppm, and further preferably 0.1 to 100 ppm.

The flavor composition of the present invention can be obtained by adding the agent for enhancing aroma intensity and/or aroma quality to any flavor which requires enhancement of aroma intensity and/or aroma quality. Flavors to which the present invention is directed are not particularly limited, and examples thereof include pineapple flavor, butter flavor, vanilla flavor, coffee flavor, chocolate flavor, caramel flavor, lemon flavor, garlic flavor, green tea flavor, rum flavor, apple flavor, grape flavor, curry flavor, soy sauce flavor, orange flavor, yogurt flavor, and mint flavor.

The flavor composition of the present invention may further comprise various compounding agents and additives used in this technical field. The compounding agents and additives which can be mixed with the flavor composition of the present invention are not particularly limited, and examples thereof include antioxidants, known preservatives and antibacterial agents, pH adjusters, and the like. A combination of any two or more of the compounding agents and additives may be used.

More specifically, the antioxidants include butylhydroxytoluene, butylhydroxyanisole, citric acid, glutathione, selenium, lycopene, vitamin A, vitamin E, vitamin C, and the like, as well as pyrrolopyrrole derivatives, free radical scavengers obtained from extracts of various plants, enzymes having antioxidant properties such as superoxide dismutase and glutathione peroxidase, and the like.

Meanwhile, the preservatives and antibacterial agents include benzoic acid, sodium benzoate, isopropyl paraoxybenzoate, isobutyl paraoxybenzoate, ethyl paraoxybenzoate, methyl paraoxybenzoate, butyl paraoxybenzoate, propyl paraoxybenzoate, sodium sulfite, sodium hypochlorite, potassium pyrosulfite, sorbic acid, potassium sorbate, sodium dehydroacetate, thujaplicin, Udo (Aralia cordata) extract, Japanese snowbell extract, Artemisia capillaris extract, oolong tea extract, milt protein extract, enzymatically hydrolyzed coix extract, tea catechins, apple polyphenols, pectin digests, chitosan, lysozymes, ε-polylysine, and the like.

The pH adjuster include adipic acid, citric acid, trisodium citrate, glucono delta-lactone, gluconic acid, potassium gluconate, sodium gluconate, DL-tartaric acid, L-tartaric acid, DL-potassium hydrogen tartrate, L-potassium hydrogen tartrate, DL-sodium tartrate, L-sodium tartrate, potassium carbonate (anhydrous), sodium hydrogen carbonate, sodium carbonate, carbon dioxide, lactic acid, sodium lactate, glacial acetic acid, disodium dihydrogen pyrophosphate, fumaric acid, monosodium fumarate, DL-malic acid, DL-sodium malate, phosphoric acid, dipotassium hydrogen phosphate, potassium dihydrogen phosphate, disodium hydrogen phosphate, sodium dihydrogen phosphate, and the like.

The present invention also provides a method for enhancing aroma intensity and/or aroma quality of a food, drink, pharmaceutical product, or oral care product, the method comprising the step of adding the flavor composition to the food, drink, pharmaceutical product, or oral care product. The concentrations of γ-aminobutyric acid and naringenin in the finished product (the food, drink, pharmaceutical product, or oral care product) can be determined, as appropriate, by those skilled in the art according to properties of the target finished product and the required effect.

The food, drink, pharmaceutical product, or oral care product to which the present invention is directed is not particularly limited, and includes various targets such as liquid, solid, semi-solid, fluid ones, as long as they can obtain benefits from the flavor improvement, to which the present invention is directed, using γ-aminobutyric acid and naringenin.

The food and drink include, but are not limited to, liquid products such as fruits juices, vegetable beverages, carbonated beverages, isotonic drinks, coffee beverages, green tea, black tea, yogurt beverages, lactic acid bacteria beverages, energy drinks, soups, and noodles soups; solid products such as candy, chewing gum, gummy candy, jelly, chocolate, cookie, ice cream, ham, sausage, and snack; and semi-solid or fluid products such as curry, stew, hashed beef stew, sauce, dipping sauce, dressing, and fresh cream. The pharmaceutical product is not particularly limited, as long as the pharmaceutical product is used with a flavor composition. Meanwhile, the oral care product includes dental powder, toothpaste, liquid toothpaste, mouthwash, and the like.

The food, drink, pharmaceutical product, or oral care product to which the present invention is directed may comprise various compounding agents and additives commonly used for foods, drinks, pharmaceutical products, or oral care products. Besides the above-described antioxidants, known preservatives and antibacterial agents, and pH adjusters, examples of the compounding agents and additives include sweeteners, acidulants, bulking agents, pigments, emulsifiers, functional substances, already-existing flavor improvement agents, milk components, nitrogen-containing compounds such as amino acids and peptides, various flavor raw materials, and the like. A combination of any two or more of these compounding agents and additives may be used.

More specifically, examples of the sweeteners include sugar, fructose, lactose, glucose, Palatinose (isomaltulose), maltose, trehalose, sorbitol, erythritol, maltitol, reduced palatinose, xylitol, lactitol starch syrup, oligosaccharides, aspartame, sucralose, acesulfame potassium, saccharin, stevia, neotame, alitame, thaumatin, neohesperidin dihydrochalcone, licorice, and the like.

The acidulants include acetic acid, lactic acid, citric acid, and the like.

The bulking agents include saccharides, polysaccharides, modified starch, casein, gelatin, carboxymethyl cellulose, lecithin, and the like.

The pigments include naturally occurring pigments, synthetic organic pigments, and the like. Specifically, the pigments include hibiscus pigment, huckleberry pigment, plum pigment, seaweed pigment, dewberry pigment, grape juice pigment, blackberry pigment, blueberry pigment, mulberry pigment, morello cherry pigment, redcurrant pigment, loganberry pigment, paprika powder, malt extract, rutin, flavonoids, red cabbage pigment, red radish pigment, adzuki bean pigment, turmeric pigment, olive tea, cowberry pigment, chlorella powder, saffron pigment, perilla pigment, strawberry pigment, chicory pigment, pecan nut pigment, red yeast rice pigment, safflower pigment, purple sweet potato pigment, lac pigment, spirulina pigment, onion pigment, tamarind pigment, capsicum pepper pigment, gardenia pigment, caramel pigment, lithospermum root pigment, rosewood pigment, krill pigment, orange pigment, carrot carotene, and the like.

Examples of the emulsifiers include fatty acid monoglycerides, fatty acid diglycerides, fatty acid triglycerides, propylene glycol fatty acid esters, sucrose fatty acid esters, polyglycerin fatty acid esters, lecithin, enzymatically modified lecithin, starch, modified starch, dextrin, sorbitan fatty acid esters, Quillaia extract, gum arabic, gum tragacanth, guar gum, karaya gum, xanthan gum, pectin, alginic acid, salts thereof, carrageenan, gelatin, casein, and the like.

The functional substances mean substances having a nutritious function or a body-conditioning function, and examples thereof include animal or vegetable fats and oils such as docosahexaenoic acid (DHA), eicosapentaenoic acid (EPA), DHA- and/or EPA-containing fish oil, linoleic acid, γ-linolenic acid, α-linolenic acid, lecithin, and diacylglycerol, and derivatives thereof; animal or plant extracts such as rosemary extract, sage extract, perilla oil, chitin, chitosan, royal jelly, and propolis; vitamins and coenzymes such as vitamin A, vitamin D, vitamin E, vitamin F, vitamin K, coenzyme Q10, and α-lipoic acid, and derivatives thereof; polyphenols such as γ-oryzanol, catechin, anthocyanin, isoflavone, rutin, chlorogenic acid, and theaflavin; dietary fibers such as indigestible dextrin; saccharides such as palatinose, xylitol, and oligosaccharides; salts such as calcium citrate malate (CCM); lactoprotein-derived substances such as casein phosphopeptide, lactoferrin, and whey peptide; lactic acid bacteria; heme iron; and the like.

The milk components include raw milk, cow's milk, whole milk powder, skim milk powder, and fresh cream, as well as lactoprotein such as casein and whey, components derived from milk of goat or sheep, degradation products thereof, and the like.

Examples of the known flavor-improving raw materials include sucralose, cyclodextrin, theanine, hesperidin glycoside, sugarcane extract, and the like.

As the various flavor raw materials, for example, naturally occurring flavors, naturally occurring essential oils, and the like, as well as various synthetic flavors can be used. These flavors are not particularly limited, as long as the flavors can be used for a food, drink, pharmaceutical product, or oral care product. Examples of the flavors include esters, aldehydes, (thio)ethers, alcohols, ketones, lactones, carboxylic acids, aliphatic hydrocarbons, nitrogen-containing heterocyclic compounds, sulfur-containing heterocyclic compounds, amines, thiols, phenols, essential oils, and the like.

Specifically, the compounds and essential oils include acetaldehyde, ethyl acetoacetate, acetophenone, anisaldehyde, amyl alcohol, α-amylcinnamaldehyde, methyl anthranilate, ionone, isoamyl alcohol, isoeugenol, isoamyl isovalerate, ethyl isovalerate, isothiocyanates, allyl isothiocyanate, isovaleraldehyde, isobutanol, isobutyraldehyde, isopropanol, isopentylamine, indole, derivatives thereof, γ-undecalactone, a mixture of 2-ethyl-3,5-dimethylpyrazine and 2-ethyl-3,6-dimethylpyrazine, ethylvanillin, 2-ethylpyrazine, 2-ethyl-3-methylpyrazine, 2-ethyl-5-methylpyrazine, 5-ethyl-2-methylpyrazine, eugenol, octanal, ethyl octanoate, isoamyl formate, geranyl formate, citronellyl formate, cinnamic acid, ethyl cinnamate, methyl cinnamate, ketones, geraniol, isoamyl acetate, ethyl acetate, geranyl acetate, cyclohexyl acetate, citronellyl acetate, cinnamyl acetate, terpinyl acetate, phenethyl acetate, butyl acetate, benzyl acetate, l-menthyl acetate, linalyl acetate, methyl salicylate, 2,3-diethyl-5-methylpyrazine, allyl cyclohexylpropionate, citral, citronellal, citronellol, 1,8-cineol, 2,3-dimethylpyrazine, 2,5-dimethylpyrazine, 2,6-dimethylpyrazine, 2,6-dimethylpyridine, cinnamyl alcohol, cinnamaldehyde, decanal, decanol, ethyl decanoate, 5,6,7,8-tetrahydroquinoxaline, 2,3,5,6-tetramethylpyrazine, terpineol, 2,3,5-trimethylpyrazine, γ-nonalactone, vanillin, para-methylacetophenone, valeraldehyde, hydroxycitronellal, hydroxycitronellal dimethyl acetal, piperidine, piperonal pyrazine, pyrrolidine, isoamyl phenylacetate, isobutyl phenylacetate, ethyl phenylacetate, 2-(3-phenylpropyl)pyridine, phenethylamine, butanol, butylamine, butyraldehyde, furfural, derivatives thereof, propanol, propionaldehyde, propionic acid, isoamyl propionate, ethyl propionate, benzyl propionate, hexanoic acid, allyl hexanoate, ethyl hexanoate, ethyl heptanoate, perillaldehyde, benzyl alcohol, benzaldehyde, 2-pentanol, 1-penten-3-ol, d-borneol, maltol, methyl N-methylanthranilate, 5-methylquinoxaline, 6-methylquinoline, 5-methyl-6,7-dihydro-5H-cyclopentapyrazine, methyl β-naphthyl ketone, 2-methylpyrazine, 2-methylbutanol, 3-methyl-2-butanol, 2-methylbutyraldehyde, 3-methyl-2-butenal, 3-methyl-2-butenol, menthol isomers such as l-menthol, butyric acid, isoamyl butyrate, ethyl butyrate, cyclohexyl butyrate, butyl butyrate, linalool, anise oil, star anise oil, bergamot oil, basil oil, West Indian bay leaf oil, galbanum oil, apple oil, apricot oil, cassia oil, camphor laurel wood oil, buchu leaf oil, cardamom seed oil, cassia bark oil, chamomile roman flower oil, cinnamon bark oil, cinnamon leaf oil, clove bud oil, cognac green oil, coriander oil, cubeb oil, caraway oil, fennel sweet oil, garlic oil, ginger oil, petitgrain oil, lemon oil, lime oil, orange oil, citrus oil, Japanese cedar (Cryptomeria japonica) wood oil, camphor laurel wood oil, citronella oil, patchouli oil, eucalyptus oil, bay oil, grapefruit oil, mandarin oil, sandalwood oil, juniper berry oil, rose oil, ylang oil, tangerine oil, geranium oil, limonene, mint oil, peppermint oil, and the like.

Hereinafter, the present invention will be described in further detail based on Examples; however, the present invention is not limited to these Examples.

### [Examples]

### Method for measuring content of component

### 1) Naringenin

Under the following conditions, samples were analyzed as they were or after diluted with water to prepare 0.1 to 100 w/v% aqueous solutions.
Measurement device: High-performance liquid chromatograph (produced by Agilent Technologies)
Column: Cosmosil 5C18-AR-II, 4.6 × 150 mm (produced by Nacalai Tesque, Inc.)
Column temperature: 40°C
Mobile phase: A: acetonitrile, B: water
A/B=10/90 (0 min) → 10/90 (5 min) → 30/70 (15 min) → 30/70 (30 min)
Flow rate: 0.8 ml/min
Detector: Ultraviolet absorption spectrophotometer (produced by Agilent Technologies)
Detection wavelength: 210 nm

### 2) γ-Aminobutyric acid

Under the following conditions, samples were analyzed as they were or after diluted with water to prepare 0.1 to 100 w/v% aqueous solutions.

### (Analytical method: OPA amino acid quantification)

Column: Cadenza CD-C18, 4.6 × 150 mm (produced by Imtakt Corp.)
Column temperature: 40°C
Mobile phase: A: 40 mM sodium dihydrogen phosphate (pH 7.8), B: acetonitrile/methanol/water = 45/45/10
Gradient: A/B = 100/0 (0 min) → 100/0 (7 min) → 80/20 (18 min) → 80/20 (20 min) → 55/45 (33.6 min) → 0/100 (35 min) → 0/100 (45 min)
Flow rate: 1.0 ml/min
Detector: Fluorescence detector (produced by Agilent Technologies)
Detection wavelength: excitation wavelength: 340 nm, fluorescence wavelength: 450 nm
   1) To a pineapple flavor, γ-aminobutyric acid (manufactured by NACALAI TESQUE, INC.) and naringenin (manufactured by LKT Laboratories, Inc.) were added in the added amounts shown in Table below to prepare flavor compositions of Example 1, Comparative Example 1-1, Comparative Example 1-2, and Comparative Example 1-3.

| | Component content (ppm) in flavor | | γ-Aminobutyric acid:Naringenin |
|---|---|---|---|
| | γ-Aminobutyric acid | Naringenin | |
| Ex. 1 | 5 | 0.1 | 50:1 |
| Comp. Ex. 1-1 | 0 | 0 | - |
| Comp. Ex. 1-2 | 0 | 0.1 | - |
| Comp. Ex. 1-3 | 5 | 0 | - |

Drinks were prepared by adding 0.1% of the prepared flavor compositions to carbonated water, and subjected to sensory evaluations. In the sensory evaluations, a drink obtained by adding only the pineapple flavor to the carbonated water was used as a control, and evaluations were made as to whether, when each of the drinks to which the flavor compositions of Example and Comparative Examples were added was ingested, each of enhancement of aroma intensity and enhancement of aroma quality was perceived or not, and enhancement of preference for each drink was perceived or not. Note that each of the drinks to which the flavor compositions of the example and the comparative examples were added was presented to the evaluators in a blinded manner.

### Number of panelists: 12

### Sensory evaluation results:

Regarding each of the items, the number of panelists who perceived an enhancement in the evaluation is shown in Table below.

| | Aroma intensity | Aroma quality | Preference |
|---|---|---|---|
| Ex. 1 | 11/12 | 11/12 | 12/12 |
| Comp. Ex. 1-1 | 0/12 | 0/12 | 0/12 |
| Comp. Ex. 1-2 | 1/12 | 0/12 | 1/12 |
| Comp. Ex. 1-3 | 1/12 | 2/12 | 1/12 |

The results of the sensory evaluations show that the addition of the predetermined amounts of γ-aminobutyric acid and naringenin to the pineapple flavor achieved enhancement of aroma intensity and enhancement of aroma quality. In addition, the flavored carbonated water was evaluated to be enhanced by the addition in terms of preference.

In each of the following sensory evaluations of Experimental Examples, a food or drink obtained by adding only a flavor to an evaluation base was used as a control, and evaluations were made as to whether, when each of foods and drinks to which flavor compositions of Example(s) and Comparative Example(s) were added to the evaluation base was ingested, each of enhancement of aroma intensity and enhancement of aroma quality was perceived or not, and enhancement of preference for each food or drink was perceived or not, relative to the control. The to-be evaluated foods or drinks to which the flavor compositions of Example and Comparative Examples were added were presented to the evaluators in a blinded manner.

### 2) To a butter flavor, γ-aminobutyric acid and naringenin were added in the added amounts shown in Table below to prepare flavor compositions of Example 2, Comparative Example 2-1, Comparative Example 2-2, and Comparative Example 2-3.

| | Component content (ppm) in flavor | | γ-Aminobutyric acid:Naringenin |
|---|---|---|---|
| | γ-Aminobutyric acid | Naringenin | |
| Ex. 2 | 5 | 0.05 | 100:1 |
| Comp. Ex. 2-1 | 0 | 0 | - |
| Comp. Ex. 2-2 | 0 | 0.1 | - |
| Comp. Ex. 2-3 | 5 | 0 | - |

Batches of cookie dough were prepared by mixing 182 g of weak flour, 96.4 g of shortening, 80.4 g of powdered sugar, 10.8 g of skim milk powder, 3.2 g of baking powder, 0.4 g of common salt, 26.8 g of water together, and further adding 0.09% of each of the flavor compositions of Example 2 and Comparative Examples 2-1, 2-2, and 2-3. Cookies obtained by baking the batches of dough under conditions of a bottom oven temperature of 140°C and a top oven temperature of 160°C were subjected to the sensory evaluations.

### Number of panelists: 10

### Sensory evaluation results:

Regarding each of the items, the number of panelists who perceived an enhancement in the evaluation is shown in Table below.

| | Aroma intensity | Aroma quality | Preference |
|---|---|---|---|
| Ex. 2 | 10/10 | 9/10 | 9/10 |
| Comp. Ex. 2-1 | 0/10 | 1/10 | 1/10 |
| Comp. Ex. 2-2 | 2/10 | 0/10 | 2/10 |
| Comp. Ex. 2-3 | 1/10 | 1/10 | 1/10 |

The results of the sensory evaluations show that the addition of the predetermined amounts of γ-aminobutyric acid and naringenin to the butter flavor achieved enhancement of aroma intensity and enhancement of aroma quality. In addition, the flavored cookies were evaluated to be enhanced by the addition in terms of preference.

3) 30 g of concentrated transparent tomato juice (LycoRed Natural Products Industries Ltd.) and 20 g of 95 vol% ethanol were mixed with each other, stirred at room temperature for 10 minutes, and then allowed to stand at room temperature for 70 hours. After the allowing to stand, the upper one of the two separated layers was recovered as a processed tomato juice product A (26.6 g). The processed tomato juice product A contained 4800 ppm of γ-aminobutyric acid and 40 ppm of naringenin.

To a vanilla flavor, the processed tomato juice product A was added at 0.1% to prepare a flavor composition of Example 3-1, the processed tomato juice product A was added at 0.001% to prepare a flavor composition of Example 3-2, and nothing was added to prepare a flavor composition of Comparative Example 3. The contents of γ-aminobutyric acid and naringenin in the flavors were as shown in Table below.

| | Component content (ppm) in flavor | | γ-Aminobutyric acid:Naringenin |
|---|---|---|---|
| | γ-Aminobutyric acid | Naringenin | |
| Ex. 3-1 | 4.8 | 0.04 | 120:1 |
| Ex. 3-2 | 0.048 | 0.0004 | 120:1 |
| Comp. Ex. 3 | 0 | 0 | - |

To vanilla ice cream, each of the flavor compositions of Example 3-1, Example 3-2, and Comparative Example 3 was added at 0.2%, and the sensory evaluations were conducted.

### Number of evaluators: 15

### Sensory evaluation results:

Regarding each of the items, the number of evaluators who noticed an enhancement in the evaluation is shown in Table below.

| | Aroma intensity | Aroma quality | Preference |
|---|---|---|---|
| Ex. 3-1 | 14/15 | 15/15 | 14/15 |
| Ex. 3-2 | 2/15 | 1/15 | 3/15 |
| Comp. Ex. 3 | 1/15 | 1/15 | 1/15 |

The results of the sensory evaluations show that the addition of the predetermined amounts of γ-aminobutyric acid and naringenin to the vanilla flavor achieved enhancement of aroma intensity and enhancement of aroma quality. In addition, the flavored vanilla ice cream was evaluated to be enhanced by the addition in terms of preference.

4) To a coffee flavor, the processed tomato juice product A added in Example 3-1 was added at 0.5% to prepare a flavor composition of Example 4-1, the processed tomato juice product A was added at 0.005% to prepare a flavor composition of Example 4-2, and nothing was added to prepare a flavor composition of Comparative Example 4. The contents of γ-aminobutyric acid and naringenin in the flavor were as shown in Table below.

| | Component content (ppm) in flavor | | γ-Aminobutyric acid:Naringenin |
|---|---|---|---|
| | γ-Aminobutyric acid | Naringenin | |
| Ex. 4-1 | 24 | 0.2 | 120:1 |
| Ex. 4-2 | 0.24 | 0.002 | 120:1 |
| Comp. Ex. 4 | 0 | 0 | - |

To coffee, each of the flavor compositions of Example 4-1, Example 4-2, and Comparative Example 4 was added at 0.1%, and the sensory evaluations were conducted.

### Number of evaluators: 15

### Sensory evaluation results:

Regarding each of the items, the number of evaluators who noticed an enhancement in the evaluation is shown in Table below.

| | Aroma intensity | Aroma quality | Preference |
|---|---|---|---|
| Ex. 4-1 | 14/15 | 14/15 | 15/15 |
| Ex. 4-2 | 1/15 | 3/15 | 2/15 |
| Comp. Ex. 4 | 3/15 | 3/15 | 0/15 |

The results of the sensory evaluations show that the addition of the predetermined amounts of γ-aminobutyric acid and naringenin to the coffee flavor achieved enhancement of aroma intensity and enhancement of aroma quality. The flavored coffee was evaluated to be enhanced by the addition in terms of preference.

5) To a chocolate flavor, γ-aminobutyric acid and naringenin were added in the added amounts shown in Table below to prepare flavor compositions of Example 5-1, Example 5-2, and Example 5-3.

| | Component content (ppm) in flavor | | γ-Aminobutyric acid:Naringenin |
|---|---|---|---|
| | γ-Aminobutyric acid | Naringenin | |
| Ex. 5-1 | 10000 | 10000 | 1:1 |
| Ex. 5-2 | 20000 | 20000 | 1:1 |
| Ex. 5-3 | 20000 | 10000 | 2:1 |

400 g of couverture chocolate was dissolved by heating with hot water at 50°C. To 100 g of the dissolved chocolate, each of the flavor compositions of Example 5-1, Example 5-2, and Example 5-3 was added at 0.1%. After the addition of each flavor composition, the chocolate was tempered by well mixing, cooling to 28°C, and heating again to 30°C. The chocolate was shaped by being poured into a mold, and cooled. Thus, chocolate for sensory evaluation was prepared. This chocolate was subjected to the sensory evaluations.

### Number of evaluators: 10

### Sensory evaluation results:

Regarding each of the items, the number of evaluators who noticed an enhancement in the evaluation is shown in Table below.

| | Aroma intensity | Aroma quality | Preference |
|---|---|---|---|
| Ex. 5-1 | 10/10 | 10/10 | 10/10 |
| Ex. 5-2 | 4/10 | 4/10 | 0/10 |
| Ex. 5-3 | 3/10 | 4/10 | 0/10 |

The results of the sensory evaluations show that the addition of the predetermined amounts of γ-aminobutyric acid and naringenin to the chocolate flavor achieved enhancement of aroma intensity and enhancement of aroma quality. In addition, the flavored chocolate was evaluated to be enhanced by the addition in terms of preference.

6) To a caramel flavor, γ-aminobutyric acid and naringenin were added in the added amounts shown in Table below to prepare flavor compositions of Example 6 and Comparative Examples 6-1, 6-2, and 6-3.

| | Component content (ppm) in flavor | | γ-Aminobutyric acid:Naringenin |
|---|---|---|---|
| | γ-Aminobutyric acid | Naringenin | |
| Ex. 6 | 1000 | 100 | 10:1 |
| Comp. Ex. 6-1 | 0 | 0 | - |
| Comp. Ex. 6-2 | 1000 | 0 | - |
| Comp. Ex. 6-3 | 0 | 100 | - |

To chocolate ice cream, each of the flavor compositions of Example 6 and Comparative Examples 6-1, 6-2, and 6-3 was added at 0.15%, and the sensory evaluations were conducted.

### Number of evaluators: 10

### Sensory evaluation results:

Regarding each of the items, the number of evaluators who noticed an enhancement in the evaluation is shown in Table below.

| | Aroma intensity | Aroma quality | Preference |
|---|---|---|---|
| Ex. 6 | 10/10 | 9/10 | 10/10 |
| Comp. Ex. 6-1 | 1/10 | 1/10 | 2/10 |
| Comp. Ex. 6-2 | 2/10 | 0/10 | 0/10 |
| Comp. Ex. 6-3 | 1/10 | 2/10 | 1/10 |

The results of the sensory evaluations show that the addition of the predetermined amounts of γ-aminobutyric acid and naringenin to the caramel flavor achieved enhancement of aroma intensity and enhancement of aroma quality. In addition, the flavored chocolate ice cream was evaluated to be enhanced by the addition in terms of preference.

7) To a lemon flavor, γ-aminobutyric acid and naringenin were added in the added amounts shown in Table below to prepare flavor compositions of Example 7-1, Comparative Example 7-1, Comparative Example 7-2, and Example 7-2.

| | Component content (ppm) in flavor | | γ-Aminobutyric acid:Naringenin |
|---|---|---|---|
| | γ-Aminobutyric acid | Naringenin | |
| Ex. 7-1 | 5 | 0.05 | 100:1 |
| Comp. Ex. 7-1 | 5 | 0 | - |
| Comp. Ex. 7-2 | 0 | 0.05 | - |
| Ex. 7-2 | 50000 | 10 | 5000:1 |

39 g of fructose, 10 g of reduced maltose syrup, 0.2 g of sodium chloride, 2 g of vitamin C, 0. 65 g of anhydrous citric acid were weighed, and the amount was adjusted to 1000 g by adding water. To this mixture used as a base, each of the flavor compositions of Example 7-1, Comparative Example 7-1, Comparative Example 7-2, and Example 7-2 was added at 0.1%, and the sensory evaluations were conducted.

### Number of evaluators: 9

### Sensory evaluation results:

Regarding each of the items, the number of evaluators who noticed an enhancement in the evaluation is shown in Table below.

| | Aroma intensity | Aroma quality | Preference |
|---|---|---|---|
| Ex. 7-1 | 9/9 | 7/9 | 9/9 |
| Comp. Ex. 7-1 | 1/9 | 3/9 | 3/9 |
| Comp. Ex. 7-2 | 2/9 | 3/9 | 0/9 |
| Ex. 7-2 | 2/9 | 3/9 | 0/9 |

The results of the sensory evaluations show that the addition of the predetermined amounts of γ-aminobutyric acid and naringenin to the lemon flavor achieved enhancement of aroma intensity and enhancement of aroma quality. In addition, the flavored drink was evaluated to be enhanced by the addition in terms of preference.

8) With 300 g of concentrated transparent tomato juice (LycoRed Natural Products Industries Ltd.), 30 g of 95 vol% ethanol was mixed. This mixture was transferred to a 500 ml flask, and heated at 90°C with stirring. When the amount of the condensate reached 30 g, the heating was stopped, and the liquid remaining unvaporized (290 g) was recovered as a processed tomato juice product B. The processed tomato juice product B contained 9100 ppm of γ-aminobutyric acid and 51 ppm of naringenin.

To a garlic flavor, the processed tomato juice product B was added at 0.2% to prepare a flavor composition of Example 8-1, the processed tomato juice product B was added at 0.002% to prepare a flavor composition of Example 8-2, and nothing was added to prepare a flavor composition of Comparative Example 8. In each of the flavors, the contents of γ-aminobutyric acid and naringenin were as shown in Table below.

| | Component content (ppm) in flavor | | γ-Aminobutyric acid:Naringenin |
|---|---|---|---|
| | γ-Aminobutyric acid | Naringenin | |
| Ex. 8-1 | 18.2 | 0.102 | 178:1 |
| Ex. 8-2 | 0.182 | 0.001 | 178:1 |
| Comp. Ex. 8 | 0 | 0 | - |

To a sauce for grilled meat, each of the flavor compositions of Example 8-1, Example 8-2, and Comparative Example 8 was added at 0.05%, and the sensory evaluations were conducted.

### Number of evaluators: 12

### Sensory evaluation results:

Regarding each of the items, the number of evaluators who noticed an enhancement in the evaluation is shown in Table below.

| | Aroma intensity | Aroma quality | Preference |
|---|---|---|---|
| Ex. 8-1 | 11/12 | 11/12 | 12/12 |
| Ex. 8-2 | 3/12 | 3/12 | 3/12 |
| Comp. Ex. 8 | 2/12 | 1/12 | 3/12 |

The results of the sensory evaluations show that the addition of the predetermined amounts of γ-aminobutyric acid and naringenin to the garlic flavor achieved enhancement of aroma intensity and enhancement of aroma quality. In addition, the flavored sauce for grilled meat was evaluated to be enhanced by the addition in terms of preference.

9) To a green tea flavor, the processed tomato juice product B was added at 0.1% to prepare a flavor composition of Example 9-1, the processed tomato juice product B was added at 0.005% to prepare a flavor composition of Example 9-2, and nothing was added to prepare a flavor composition of Comparative Example 9. The contents of γ-aminobutyric acid and naringenin in each of the flavor were as shown in Table below.

| | Component content (ppm) in flavor | | γ-Aminobutyric acid:Naringenin |
|---|---|---|---|
| | γ-Aminobutyric acid | Naringenin | |
| Ex. 9-1 | 9.1 | 0.051 | 178:1 |
| Ex. 9-2 | 0.455 | 0.0026 | 178:1 |
| Comp. Ex. 9 | 0 | 0 | - |

To green tea, each of the flavor compositions of Example 9-1, Example 9-2, and Comparative Example 9 was added at 0.04%, and the sensory evaluations were conducted.

### Number of evaluators: 10

### Sensory evaluation results:

Regarding each of the items, the number of evaluators who noticed an enhancement in the evaluation is shown in Table below.

| | Aroma intensity | Aroma quality | Preference |
|---|---|---|---|
| Ex. 9-1 | 8/10 | 10/10 | 10/10 |
| Ex. 9-2 | 2/10 | 2/10 | 1/10 |
| Comp. Ex. 9 | 2/10 | 0/10 | 0/10 |

The results of the sensory evaluations show that the addition of the predetermined amounts of γ-aminobutyric acid and naringenin to the green tea flavor achieved enhancement of aroma intensity and enhancement of aroma quality. In addition, the flavored green tea was evaluated to be enhanced by the addition in terms of preference.

10) To a rum flavor, γ-aminobutyric acid and naringenin were added in the added amounts shown in Table below to prepare flavor compositions of Example 10 and Comparative Examples 10-1, 10-2, and 10-3. The contents of γ-aminobutyric acid and naringenin in each of the flavors were as shown in Table below.

| | Component content (ppm) in flavor | | γ-Aminobutyric acid:Naringenin |
|---|---|---|---|
| | γ-Aminobutyric acid | Naringenin | |
| Ex. 10 | 10 | 1 | 10:1 |
| Comp. Ex. 10-1 | 0 | 0 | - |
| Comp. Ex. 10-2 | 10 | 0 | - |
| Comp. Ex. 10-3 | 0 | 1 | - |

To vanilla ice cream, each of the flavor compositions of Example 10 and Comparative Examples 10-1, 10-2, and 10-3 was added at 0.05%, and the sensory evaluations were conducted.

### Number of evaluators: 10

### Sensory evaluation results:

Regarding each of the items, the number of evaluators who noticed an enhancement in the evaluation is shown in Table below.

| | Aroma intensity | Aroma quality | Preference |
|---|---|---|---|
| Ex. 10 | 8/10 | 10/10 | 8/10 |
| Comp. Ex. 10-1 | 1/10 | 2/10 | 2/10 |
| Comp. Ex. 10-2 | 2/10 | 3/10 | 0/10 |
| Comp. Ex. 10-3 | 1/10 | 3/10 | 0/10 |

The results of the sensory evaluations show that the addition of the predetermined amounts of γ-aminobutyric acid and naringenin to the rum flavor achieved enhancement of aroma intensity and enhancement of aroma quality. In addition, the flavored vanilla ice cream was evaluated to be enhanced by the addition in terms of preference.

11) To an apple flavor, γ-aminobutyric acid and naringenin were added in the added amounts shown in Table below to prepare flavor compositions of Example 11 and Comparative Examples 11-1, 11-2, and 11-3. The contents of γ-aminobutyric acid and naringenin in each of the flavors were as shown in Table below.

| | Component content (ppm) in flavor | | γ-Aminobutyric acid:Naringenin |
|---|---|---|---|
| | γ-Aminobutyric acid | Naringenin | |
| Ex. 11 | 100 | 100 | 1:1 |
| Comp. Ex. 11-1 | 0 | 0 | - |
| Comp. Ex. 11-2 | 100 | 0 | - |
| Comp. Ex. 11-3 | 0 | 100 | - |

To 400 g of Palatinit (registered trademark), 142.5 g of reduced starch saccharide and 150 g of water were added, and the material was completely dissolved with heating. After heating to 170°C, the mixture was cooled. When the temperature reached 150°C or below, 0.5 g of Sunett (registered trademark) D and 0.25 g of Mirasee (registered trademark) 200, and 1 g of the flavor composition of Example 11, Comparative Example 11-1, Comparative Example 11-2, or Comparative Example 11-3 was added, and the mixture was shaped by being poured into molds. Thus, candies for sensory evaluation were prepared. The candies were subjected to the sensory evaluations.

### Number of evaluators: 12

### Sensory evaluation results:

Regarding each of the items, the number of evaluators who noticed an enhancement in the evaluation is shown in Table below.

| | Aroma intensity | Aroma quality | Preference |
|---|---|---|---|
| Ex. 11 | 12/12 | 10/12 | 11/12 |
| Comp. Ex. 11-1 | 1/12 | 2/12 | 2/12 |
| Comp. Ex. 11-2 | 2/12 | 2/12 | 1/12 |
| Comp. Ex. 11-3 | 1/12 | 3/12 | 2/12 |

The results of the sensory evaluations show that the addition of the predetermined amounts of γ-aminobutyric acid and naringenin to the apple flavor achieved enhancement of aroma intensity and enhancement of aroma quality. In addition, the flavored candy was evaluated to be enhanced by the addition in terms of preference.

12) To a grape flavor, γ-aminobutyric acid and naringenin were added in the added amounts shown in Table below to prepare flavor compositions of Example 12 and Comparative Examples 12-1, 12-2, and 12-3. The contents of γ-aminobutyric acid and naringenin in each of the flavors were as shown in Table below.

| | Component content (ppm) in flavor | | γ-Aminobutyric acid:Naringenin |
|---|---|---|---|
| | γ-Aminobutyric acid | Naringenin | |
| Ex. 12 | 30 | 10 | 3:1 |
| Comp. Ex. 12-1 | 0 | 0 | - |
| Comp. Ex. 12-2 | 30 | 0 | - |
| Comp. Ex. 12-3 | 0 | 10 | - |

To 400 g of Palatinit (registered trademark), 142.5 g of reduced starch saccharide and 150 g of water were added, and the material was completely dissolved with heating. The material was heated to 170°C, and then cooled. When the temperature reached 150°C or below, 0.5 g of Sunett (registered trademark) D, 0.25 g of Mirasee (registered trademark) 200, and 1 g of the flavor composition of Example 11, Comparative Example 11-1, Comparative Example 11-2, or Comparative Example 11-3 was added, and the mixture was shaped by pouring into molds. Thus, candies for sensory evaluation were prepared. The candies were subjected to the sensory evaluations.

### Number of evaluators: 12

### Sensory evaluation results:

Regarding each of the items, the number of evaluators who noticed an enhancement in the evaluation is shown in Table below.

| | Aroma intensity | Aroma quality | Preference |
|---|---|---|---|
| Ex. 12 | 10/12 | 12/12 | 9/12 |
| Comp. Ex. 12-1 | 2/12 | 0/12 | 4/12 |
| Comp. Ex. 12-2 | 3/12 | 1/12 | 2/12 |
| Comp. Ex. 12-3 | 3/12 | 1/12 | 1/12 |

The results of the sensory evaluations show that the addition of the predetermined amounts of γ-aminobutyric acid and naringenin to the grape flavor achieved enhancement of aroma intensity and enhancement of aroma quality. In addition, the flavored candy was evaluated to be enhanced by the addition in terms of preference.

13) To a curry flavor, the processed tomato juice product A was added at 1% to prepare a flavor composition of Example 13-1, the processed tomato juice product A was added at 0.001% to prepare a flavor composition of Example 13-2, and nothing was added to prepare a flavor composition of Comparative Example 13. The contents of γ-aminobutyric acid and naringenin in each of the flavors were as shown in Table below.

| | Component content (ppm) in flavor | | γ-Aminobutyric acid:Naringenin |
|---|---|---|---|
| | γ-Aminobutyric acid | Naringenin | |
| Ex. 13-1 | 48 | 0.4 | 120:1 |
| Ex. 13-2 | 0.048 | 0.0004 | 120:1 |
| Comp. Ex. 13 | 0 | 0 | - |

To 500 g of water, 60 g of solid curry roux was added and dissolved by heating to prepare a curry sauce. To this curry sauce, each of the flavor compositions of Example 13-1, Example 13-2, and Comparative Example 13 was added at 1%, and the sensory evaluations were conducted.

### Number of evaluators: 9

### Sensory evaluation results:

Regarding each of the items, the number of evaluators who noticed an enhancement in the evaluation is shown in Table below.

| | Aroma intensity | Aroma quality | Preference |
|---|---|---|---|
| Ex. 13-1 | 8/9 | 9/9 | 8/9 |
| Ex. 13-2 | 2/9 | 1/9 | 2/9 |
| Comp. Ex. 13 | 2/9 | 1/9 | 1/9 |

The results of the sensory evaluations show that the addition of the predetermined amounts of γ-aminobutyric acid and naringenin to the curry flavor achieved enhancement of aroma intensity and enhancement of aroma quality. In addition, the flavored curry sauce was evaluated to be enhanced by the addition in terms of preference.

14) To a soy sauce flavor, the processed tomato juice product B was added at 0.3% to prepare a flavor composition of Example 14-1, the processed tomato juice product B was added at 0.001% to prepare a flavor composition of Example 14-2, and nothing was added to prepare a flavor composition of Comparative Example 14. The contents of γ-aminobutyric acid and naringenin in each of the flavors were as shown in Table below.

| | Component content (ppm) in flavor | | γ-Aminobutyric acid:Naringenin |
|---|---|---|---|
| | γ-Aminobutyric acid | Naringenin | |
| Ex. 14-1 | 27.3 | 0.153 | 178:1 |
| Ex. 14-2 | 0.091 | 0.00051 | 178:1 |
| Comp. Ex. 14 | 0 | 0 | - |

600 g of soy sauce, 450 g of sugar, 63 g of common salt, 300 g of dried bonito extract, and 45 g of potassium chloride were mixed together, and heated with hot water to 90°C to prepare a three-times concentrated noodle soup. This noodle soup was diluted 3 times with water. Then, each of the flavor compositions of Example 14 and Comparative Examples 14-1 and 14-2 was added at 0.5% thereto, and the sensory evaluations were conducted.

### Number of evaluators: 11

### Sensory evaluation results:

Regarding each of the items, the number of evaluators who noticed an enhancement in the evaluation is shown in Table below.

| | Aroma intensity | Aroma quality | Preference |
|---|---|---|---|
| Ex. 14 | 11/11 | 10/11 | 10/11 |
| Comp. Ex. 14-1 | 1/11 | 0/11 | 2/11 |
| Comp. Ex. 14-2 | 2/11 | 1/11 | 0/11 |

The results of the sensory evaluations show that the addition of the predetermined amounts of γ-aminobutyric acid and naringenin to the soy sauce flavor achieved enhancement of aroma intensity and enhancement of aroma quality. In addition, the flavored noodle soup was evaluated to be enhanced by the addition in terms of preference.

15) To an orange flavor, γ-aminobutyric acid and naringenin were added in the added amounts shown in Table below to prepare flavor compositions of Example 15, Comparative Example 15-1, and Comparative Example 15-2. The contents of γ-aminobutyric acid and naringenin in each of the flavors were as shown in Table below.

| | Component content (ppm) in flavor | | γ-Aminobutyric acid:Naringenin |
|---|---|---|---|
| | γ-Aminobutyric acid | Naringenin | |
| Ex. 15 | 1 | 100 | 1:100 |
| Comp. Ex. 15-1 | 1 | 0 | - |
| Comp. Ex. 15-2 | 0 | 100 | - |

A drink was prepared whose amount was adjusted to 1000 g by adding water to 2.5 g of anhydrous citric acid, 1 g of citric acid, 0.4 g of Rebaudio J-100, and 0.2 g vitamin C. To this drink, each of the flavor compositions of Example 15 and Comparative Example 15-1 and 15-2 was added at 0.1%, and the sensory evaluations were conducted.

### Number of evaluators: 16

### Sensory evaluation results:

Regarding each of the items, the number of evaluators who noticed an enhancement in the evaluation is shown in Table below.

| | Aroma intensity | Aroma quality | Preference |
|---|---|---|---|
| Ex. 15 | 14/16 | 16/16 | 15/16 |
| Comp. Ex. 15-1 | 1/16 | 2/16 | 1/16 |
| Comp. Ex. 15-2 | 2/16 | 3/16 | 3/16 |

The results of the sensory evaluations show that the addition of the predetermined amounts of γ-aminobutyric acid and naringenin to the orange flavor achieved enhancement of aroma intensity and enhancement of aroma quality. In addition, the flavored drink was evaluated to be enhanced by the addition in terms of preference.

16) To an apple flavor, γ-aminobutyric acid and naringenin were added in the added amounts shown in Table below to prepare flavor compositions of Example 16 and Comparative Examples 16-1 and 16-2. The contents of γ-aminobutyric acid and naringenin in each of the flavors were as shown in Table below.

| | Component content (ppm) in flavor | | γ-Aminobutyric acid:Naringenin |
|---|---|---|---|
| | γ-Aminobutyric acid | Naringenin | |
| Ex. 16 | 0.1 | 1000 | 1:10000 |
| Comp. Ex. 16-1 | 0.1 | 0 | - |
| Comp. Ex. 16-2 | 0 | 1000 | - |

Each of the flavor compositions of Example 16 and Comparative Examples 16-1 and 16-2 was added at 0.1% to water, and the sensory evaluations were conducted.

### Number of evaluators: 15

### Sensory evaluation results:

Regarding each of the items, the number of evaluators who noticed an enhancement in the evaluation is shown in Table below.

| | Aroma intensity | Aroma quality | Preference |
|---|---|---|---|
| Ex. 16 | 14/15 | 15/15 | 13/15 |
| Comp. Ex. 16-1 | 1/15 | 2/15 | 1/15 |
| Comp. Ex. 16-2 | 2/15 | 3/15 | 2/15 |

The results of the sensory evaluations show that the addition of the predetermined amounts of γ-aminobutyric acid and naringenin to the apple flavor achieved enhancement of aroma intensity and enhancement of aroma quality. In addition, the flavored water was evaluated to be enhanced by the addition in terms of preference.

17) To a yogurt flavor, the processed tomato juice product B was added at 0.2% to prepare a flavor composition of Example 17-1, the processed tomato juice product B was added at 0.001% to prepare a flavor composition of Example 17-2, and nothing was added to prepare a flavor composition of Comparative Example 17. The contents of γ-aminobutyric acid and naringenin in each of the flavors were as shown in Table below.

| | Component content (ppm) in flavor | | γ-Aminobutyric acid:Naringenin |
|---|---|---|---|
| | γ-Aminobutyric acid | Naringenin | |
| Ex. 17 | 18.2 | 0.102 | 178:1 |
| Comp. Ex. 17-1 | 0.091 | 0.0051 | 178:1 |
| Comp. Ex. 17-2 | 0 | 0 | - |

1070 g of a drink was prepared by well mixing 15 g of skim milk powder, 100 g of sugar, 4 g of citric acid, and 956 g of water together. To this drink, each of the flavor compositions of Example 17-1, Example 17-2, and Comparative Example 17 was added at 0.1%, and the sensory evaluations were conducted.

### Number of evaluators: 17

### Sensory evaluation results:

Regarding each of the items, the number of evaluators who noticed an enhancement in the evaluation is shown in Table below.

| | Aroma intensity | Aroma quality | Preference |
|---|---|---|---|
| Ex. 17 | 17/17 | 15/17 | 15/17 |
| Comp. Ex. 17-1 | 3/17 | 3/17 | 4/17 |
| Comp. Ex. 17-2 | 2/17 | 2/17 | 2/17 |

The results of the sensory evaluations show that the addition of the predetermined amounts of γ-aminobutyric acid and naringenin to the yogurt flavor achieved enhancement of aroma intensity and enhancement of aroma quality. In addition, the flavored drink was evaluated to be enhanced by the addition in terms of preference.

18) To a mint flavor, γ-aminobutyric acid and naringenin were added in the added amounts shown in Table below to prepare flavor compositions of Example 17, Comparative Example 17-1, Comparative Example 17-2, and Comparative Example 17-3.

| | Component content (ppm) in flavor | | γ-Aminobutyric acid:Naringenin |
|---|---|---|---|
| | γ-Aminobutyric acid | Naringenin | |
| Ex. 18 | 10 | 10 | 1:1 |
| Comp. Ex. 18-1 | 0 | 0 | - |
| Comp. Ex. 18-2 | 10 | 0 | - |
| Comp. Ex. 18-3 | 0 | 10 | - |

40 g of 95 vol% ethyl alcohol and 8 g of oxyethylene-hardened castor oil were mixed with each other, and further 0.4 g of each of the flavor compositions of Example 17, Comparative Example 17-1, Comparative Example 17-2, and Comparative Example 17-3 was added. Subsequently, 0.08 g of sodium saccharin, 40 g of glycerin, 0.2 g of sodium benzoate, and 311.32 g of purified water were added. The materials were well mixed to obtain 400 g of each of mouthwashes containing the flavor compositions of Example 17, Comparative Example 17-1, Comparative Example 17-2, and Comparative Example 17-3. Panelists held 30 ml of each of the mouthwashes in their mouths, and rinsed the inside of their mouths for 30 seconds, and spit out the mouthwash. After that, the aroma in the oral cavity was sensorily evaluated.

### Number of panelists: 11

### Sensory evaluation results:

Regarding each of the items, the number of panelists who noticed the enhancement in the evaluation is shown in Table below.

| | Aroma intensity | Aroma quality | Preference |
|---|---|---|---|
| Ex. 18 | 10/11 | 10/11 | 11/11 |
| Comp. Ex. 18-1 | 3/11 | 3/11 | 4/11 |
| Comp. Ex. 18-2 | 3/11 | 0/11 | 2/11 |
| Comp. Ex. 18-3 | 0/11 | 2/11 | 2/11 |

The results of the sensory evaluations show that the addition of the predetermined amounts of γ-aminobutyric acid and naringenin to the mint flavor achieved enhancement of aroma intensity and enhancement of aroma quality. In addition, the results show that the aroma in the oral cavity treated with the flavored mouthwash was enhanced by the addition in terms of preference.

19) To a mint flavor, the processed tomato juice product A was added at 0.5% to prepare a flavor composition of Example 19-1, the processed tomato juice product A was added at 0.001% to prepare a flavor composition of Example 19-2, and nothing was added to prepare a flavor composition of Comparative Example 19. The contents of γ-aminobutyric acid and naringenin in each of the flavors were as shown in Table below.

| | Component content (ppm) in flavor | | γ-Aminobutyric acid:Naringenin |
|---|---|---|---|
| | γ-Aminobutyric acid | Naringenin | |
| Ex. 19-1 | 24 | 0.2 | 120:1 |
| Ex. 19-2 | 0.048 | 0.0004 | 120:1 |
| Comp. Ex. 19 | 0 | 0 | - |

10 g of calcium carbonate, 2 g of sodium lauryl sulfate, 0.5 g of carboxymethyl cellulose sodium salt, 0.02 g of sodium saccharin, 10 g of sodium benzoate, and 76.48 g of glycerin were mixed together, and further 1 g of each of the flavor compositions of Example 19-1, Example 19-2, and Comparative Example 19 was added. These materials were well mixed to obtain 100 g of each of dentifrices containing the flavor compositions of Example 19-1, Example 19-2, and Comparative Example 19. 0.5 g of each of the dentifrices was applied to a brush, and the inside of the mouth was washed by brushing 200 times or more for 2 minutes, and rinsed with water. Then, the aroma perceived in the oral cavity was sensorily evaluated.

### Number of panelists: 12

### Sensory evaluation results:

Regarding each of the items, the number of panelists who noticed the enhancement in the evaluation is shown in Table below.

| | Aroma intensity | Aroma quality | Preference |
|---|---|---|---|
| Ex. 19-1 | 12/12 | 11/12 | 10/12 |
| Ex. 19-2 | 0/12 | 2/12 | 4/12 |
| Comp. Ex. 19 | 2/12 | 2/12 | 2/12 |

The results of the sensory evaluations show that the addition of the predetermined amounts of γ-aminobutyric acid and naringenin to the mint flavor achieved enhancement of aroma intensity and enhancement of aroma quality. In addition, the results show that the aroma in the oral cavity after treated with the flavored dentifrice was enhanced by the addition in terms of preference.

## Claims

1. An agent for enhancing aroma intensity and/or aroma quality of a flavor composition, the agent comprising γ-aminobutyric acid and naringenin.

2. The agent for enhancing aroma intensity and/or aroma quality according to claim 1, wherein
the weight ratio of γ-aminobutyric acid to naringenin is 1:10000 to 100000:1.

3. The agent for enhancing aroma intensity and/or aroma quality according to claim 1 or 2, wherein
the γ-aminobutyric acid and the naringenin are originated from a processed tomato product.

4. A method for enhancing aroma intensity and/or aroma quality of a flavor composition, the method comprising
adding γ-aminobutyric acid and naringenin to the flavor composition.

5. The method according to claim 4, wherein
the γ-aminobutyric acid and the naringenin are added to the flavor composition such that a weight ratio of the γ-aminobutyric acid to the naringenin is from 1:10000 to 100000:1.

6. A flavor composition, which comprises the agent for enhancing aroma intensity and/or aroma quality according to any one of claims 1 to 3, or which has aroma intensity and/or aroma quality enhanced by the method according to claim 4 or 5.

7. The flavor composition according to claim 6, wherein
the content of the γ-aminobutyric acid is 0.1 to 10000 ppm, and
the content of the naringenin is 0.01 to 10000 ppm.

8. A method for enhancing aroma intensity and/or aroma quality of a food, drink, pharmaceutical product, or oral care product, the method comprising the step of adding the flavor composition according to claim 6 or 7 to the food, drink, pharmaceutical product, or oral care product.

9. A food, drink, pharmaceutical product, or oral care product comprising the flavor composition according to claim 6 or 7.
